(19) Europäisches Patentamt / European Patent Office / Office européen des brevets

(11) **EP 3 008 050 B1**

(12) **EUROPÄISCHE PATENTSCHRIFT**

(45) Veröffentlichungstag und Bekanntmachung des Hinweises auf die Patenterteilung:
**14.12.2016 Patentblatt 2016/50**

(51) Int Cl.:
***C07D 307/89*** (2006.01)   ***B01J 8/04*** (2006.01)
***B01J 8/06*** (2006.01)

(21) Anmeldenummer: **15719660.1**

(22) Anmeldetag: **23.04.2015**

(86) Internationale Anmeldenummer:
**PCT/EP2015/058849**

(87) Internationale Veröffentlichungsnummer:
**WO 2015/162227 (29.10.2015 Gazette 2015/43)**

(54) **KATALYSATORANORDNUNG MIT OPTIMIERTEM LÜCKENGRAD ZUR HERSTELLUNG VON PHTHALSÄUREANHYDRID**

CATALYST ARRANGEMENT WITH OPTIMIZED VOID FRACTION FOR THE PRODUCTION OF PHTHALIC ACID ANHYDRIDE

DISPOSITIF CATALYTIQUE PRÉSENTANT UN DEGRÉ DE VIDE OPTIMISÉ POUR LA PRODUCTION D'ANHYDRIDE D'ACIDE PHTALIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.04.2014 DE 102014005940**

(43) Veröffentlichungstag der Anmeldung:
**20.04.2016 Patentblatt 2016/16**

(73) Patentinhaber: **Clariant International Ltd 4132 Muttenz (CH)**

(72) Erfinder:
• **RICHTER, Oliver 82110 Germering (DE)**
• **MESTL, Gerhard 80935 München (DE)**
• **LESSER, David 81543 München (DE)**
• **MARX, Robert 81539 München (DE)**
• **FROMM, Nadine 93109 Großkarolinenfeld (DE)**
• **SCHULZ, Felix 80469 München (DE)**
• **SCHINKE, Peter 80804 München (DE)**
• **PITSCHI, Werner 83052 Bruckmühl (DE)**

(74) Vertreter: **Silber, Anton et al Clariant Produkte (Deutschland) GmbH Patent & License Management Lenbachplatz 6 80333 München (DE)**

(56) Entgegenhaltungen:
**EP-A1- 2 363 203    WO-A1-2011/032658**

**Beschreibung**

Die Erfindung betrifft eine Katalysatoranordnung zur Herstellung von Phthalsäureanhydrid durch Gasphasenoxidation aromatischer Kohlenwasserstoffe, umfassend einen Reaktor mit einer Gaseintrittsseite für ein Eduktgas und einer Gasaustrittsseite für ein Produktgas, sowie einer ersten Katalysatorlage aus Katalysatorkörpern und mindestens einer zweiten Katalysatorlage aus Katalysatorkörpern, wobei die erste Katalysatorlage an der Gaseintrittsseite und die zweite Katalysatorlage nachfolgend zur ersten Katalysatorlage in Gasdurchflussrichtung angeordnet ist und wobei die Länge der ersten Katalysatorlage in Gasdurchflussrichtung kleiner ist, als die Länge in Gasdurchflussrichtung der zweiten Katalysatorlage, dadurch gekennzeichnet, dass die erste Katalysatorlage im Vergleich zur zweiten Katalysatorlage einen höheren Lückengrad aufweist.

Die großtechnische Produktion von Phthalsäureanhydrid erfolgt durch katalytische Gasphasenoxidation von ortho-Xylol und / oder Naphthalin. Zu diesem Zweck wird ein für die Reaktion geeigneter Katalysator, im Allgemeinen ein vanadiumhaltiger Katalysator, in einem Reaktor bereitgestellt und ein Reaktionsgas über den Katalysator geleitet. Vorzugsweise verwendet man als Reaktor einen sogenannten Rohrbündelreaktor, in dem eine Vielzahl von Rohren parallel angeordnet sind und die von einem Kühlmittel umströmt werden. Als Kühlmittel verwendet man im Allgemeinen eine Salzschmelze, beispielsweise ein eutektisches Gemisch aus $NaNO_2$ und $KNO_3$. Der Katalysator wird in Form von Katalysatorkörpern in die Rohre eingefüllt. Im einfachsten Fall verwendet man eine homogene Schüttung. Über die Schüttung wird dann ein Reaktionsgas geleitet, welches ein Gemisch aus einem sauerstoff-haltigen Gas, meist Luft, und dem zu oxidierenden Kohlenwasserstoff, meist ortho-Xylol oder Naphthalin, enthält.

Heutzutage werden industrielle Phthalsäureanhydrid-Katalysatoren auf Basis von $V_2O_5$-$TiO_2$ -haltigen Aktivmassen verwendet, die als Schicht auf Trägerringen - meist Steatit - aufgetragen werden. Die Oxidation des Kohlenwasserstoffs ist stark exotherm, so dass insbesondere im Bereich des Reaktoreingangs eine starke Wärmeentwicklung zu beobachten ist, die zur Totaloxidation des Kohlenwasserstoffs und zur Deaktivierung des Katalysators führen kann. Um den damit einhergehenden Produktivitätsverlust zu vermeiden, ist man dazu übergegangen, strukturierte Katalysator-Schüttungen, d.h. Katalysatoranordnungen, zu verwenden, wobei in den Rohren Lagen aus Katalysatoren unterschiedlicher Aktivität übereinander angeordnet sind. Gegenwärtig werden meist drei- oder vierlagige Katalysator-Schüttungen verwendet, wobei auf der Seite des Reaktoreingangs eine erste Katalysatorlage mit relativ niedriger Aktivität angeordnet ist, auf welche dann Katalysatorlagen mit schrittweise erhöhter Aktivität folgen. Die Katalysatorlage mit der höchsten Aktivität ist also auf der Seite des Reaktorausgangs angeordnet. Solche Systeme sind beispielsweise aus den Schriften WO 99/61434 A1, WO99/61433 A1 oder WO 2004/103944 A1 bekannt.

In jüngerer Zeit ist man dazu übergegangen, vermehrt Katalysatorsysteme mit vier oder mehr Lagen zu verwenden, wobei an der Reaktoreingangsseite zunächst eine relativ kurze Lage eines Katalysators höherer Aktivität angeordnet ist. An dieser Lage höherer Aktivität schließt sich eine Lage mit niedriger Aktivität an, die von weiteren Lagen gefolgt wird, bei denen sich die Katalysatoraktivität wieder erhöht. Solche Katalysatorsysteme sind beispielsweise aus den Schriften WO 2007/134849 A1 und WO 2011/032658 A1 bekannt.

WO 2006/092304 A1 beschreibt die Verwendung eines Katalysators enthaltend mindestens eine erste zur Gaseintrittsseite hin gelegene Katalysatorlage, eine zweite, näher zur Gasaustrittseite hin gelegene Katalysatorlage und eine dritte, noch näher zur oder an der Gasaustrittsseite hin gelegene Katalysatorlage zur Herstellung von Phthalsäureanhydrid durch Gasphasenoxidation von o-Xylol und/oder Naphthalin, wobei die Katalysatorlagen vorzugsweise jeweils eine Aktivmasse enthaltend $TiO_2$ aufweisen, dadurch gekennzeichnet, dass die Katalysatoraktivität der ersten Katalysatorlage höher ist, als die Katalysatoraktivität der zweiten Katalysatorlage. Die Aktivität der ersten Katalysatorlage kann durch alle dem Fachmann geläufigen Maßnahmen so eingestellt werden, dass sie höher liegt als die Aktivität der nachfolgenden zweiten Katalysatorlage. Nach einer bevorzugten Ausführungsform kann die erhöhte Aktivität in der ersten Katalysatorlage beispielsweise erzielt werden durch eine Erhöhung der Schüttdichte in der ersten Katalysatorlage, z.B. durch Einsatz einer anderen (Ring)geometrie des verwendeten inerten Formkörpers.

WO 2008/077791 A1 beschreibt ein Verfahren zur Gasphasenoxidation, bei dem man einen gasförmigen Strom, der einen aromatischen Kohlenwasserstoff und molekularen Sauerstoff umfasst, durch zwei oder mehrere Katalysatorlagen leitet. Ferner betrifft diese Offenlegungsschrift ein Katalysatorsystem zur Gasphasenreaktion unter Verwendung einer Vorlage. Das Produkt aus Durchmesser mal Höhe, oder das Volumen der vorgelagerten Inert- und/oder Katalysatoren-Ringe, ist kleiner als mindestens einer der folgenden Katalysatorlagen oder der Quotient aus Oberfläche pro Volumen der vorgelagerten Inert- und/oder Katalysatoren-Ringe ist größer als mindestens einer der folgenden Katalysatorlagen.

EP 0985648 A1 betrifft die Gasphasenoxidation von Kohlenwasserstoffen, bei der eine gasförmige Mischung umfassend ein molekularen Sauerstoff enthaltendes Gas und Kohlenwasserstoffe, die Substituenten enthalten können, durch ein Katalysatorfestbett geleitet wird und stellt ein Verfahren zur Gasphasenoxidation zur Verfügung, das durchgeführt wird, indem eine gasförmige Mischung von Rohmaterialien durch ein Katalysatorfestbett geleitet wird, in dem sich der Hohlraumanteil der Katalysatorschichten in einem oder mehreren Schritten in Strömungsrichtung entlang des Flusses der gasförmigen Mischung der Rohmaterialien erhöht.

Bei der Oxidation von ortho-Xylol oder Naphthalin entstehen neben dem Wertprodukt Phthalsäureanhydrid noch eine Reihe unerwünschter Nebenprodukte, die durch unvollständige Oxidation oder durch Überoxidation entstehen. Nebenprodukte, die durch unvollständige Oxidation entstehen, sind hauptsächlich ortho-Tolylaldehyd und Phthalid. Nebenprodukte, die durch Überoxidation entstehen, sind hauptsächlich Kohlenmonoxid, Kohlendioxid und Maleinsäureanhydrid, neben geringeren Mengen an Benzoesäure und Citraconsäureanhydrid. Gewünscht wird eine möglichst hohe Selektivität der Oxidation zu Phthalsäureanhydrid bei möglichst geringen Anteilen von Nebenprodukten im Endprodukt, darunter insbesondere Maleinsäureanhydrid, bei gleichzeitigem hohem Umsatz des Ausgangsprodukts.

Gegenwärtig werden molare Selektivitäten an Phthalsäureanhydrid von bis zu 83 mol.-% erreicht. Um die Selektivität der Oxidation von ortho-Xylol bzw. Naphthalin zu Phthalsäureanhydrid weiter zu erhöhen, können verschiedene Parameter des Katalysatorsystems variiert werden. So kann die Zusammensetzung des Katalysators variiert werden oder auch die Eigenschaften der Schüttung des Katalysators. Dazu kann beispielsweise die Anordnung und die Länge der einzelnen Katalysatorlagen variiert werden.

Der Erfindung liegt die Aufgabe zugrunde, eine Katalysatoranordnung zur Herstellung von Phthalsäureanhydrid durch Gasphasenoxidation aromatischer Kohlenwasserstoffe bereitzustellen, die im Vergleich zu den aus dem Stand der Technik bekannten Katalysatoranordnungen eine erhöhte Ausbeute an Phthalsäureanhydrid ermöglicht und den Erhalt von roh-Phthalsäureanhydrid mit einem höheren Reinheitsgrad.

Die Aufgabe der Erfindung wird gelöst mit einer Katalysatoranordnung zur Herstellung von Phthalsäureanhydrid durch Gasphasenoxidation aromatischer Kohlenwasserstoffe, umfassend einen Reaktor mit einer Gaseintrittsseite für ein Eduktgas und einer Gasaustrittsseite für ein Produktgas, sowie einer ersten Katalysatorlage aus Katalysatorkörpern und mindestens einer zweiten Katalysatorlage aus Katalysatorkörpern, wobei die erste Katalysatorlage an der Gaseintrittsseite und die zweite Katalysatorlage nachfolgend zur ersten Katalysatorlage in Gasdurchflussrichtung angeordnet ist und wobei die Länge der ersten Katalysatorlage in Gasdurchflussrichtung kleiner ist als die Länge in Gasdurchflussrichtung der zweiten Katalysatorlage, dadurch gekennzeichnet, dass die erste Katalysatorlage im Vergleich zur zweiten Katalysatorlage einen höheren Lückengrad aufweist.

Das erfindungsgemäße Verfahren wird in einem oder mehreren Reaktor(en) durchgeführt, wobei der oder die Reaktor(en) vorzugweise röhrenförmig sind (Reaktorrohre oder Rohre). Die Öffnungen des Reaktors, typischerweise die beiden Öffnungen des Reaktorrohrs, bilden einen Reaktoreingang für das Eduktgas sowie einen Reaktorausgang für das Produktgas, so dass eine Gaseintrittsseite, eine Gasaustrittseite und eine Gasdurchflussrichtung vorhanden sind. Der Reaktor wird z.B. in einem Salzbad auf eine Temperatur von zwischen 340°C und 450°C erhitzt, wobei sich durch die exotherme Reakt ion und durch die eventuell vorhandenen verschiedenen Katalysatorlagen ein Temperaturprofil ausbildet.

Der vorzugsweise röhrenförmige Reaktor weist jeweils einen Durchmesser (D) auf, wobei hier auf den Innendurchmesser des Reaktors Bezug genommen wird. Bevorzugt wird ein Durchmesser (D) des Reaktors im Bereich von 10 bis 50 mm, weiter bevorzugt 20 bis 40 mm gewählt. Die röhrenförmigen Reaktoren weisen eine Rohrlänge (L) auf, welche ebenfalls in üblichen Bereichen gewählt wird, so z.B. 2 bis 5 m. Die Rohrlänge L entspricht dabei dem Anteil der Länge des Reaktorrohrs, welcher mit den Katalysatorkörpern gefüllt ist.

Der Reaktor wird jeweils mit Katalysatorkörpern gefüllt, die die Gasphasenoxidation des ortho-Xylols oder Naphthalins katalysieren. Die Katalysatorkörper bestehen üblicherweise aus einem inerten Trägerkörper, der mit einer vanadiumhaltigen Aktivmasse, d.h. der katalytisch aktive Masse, beladen ist. Erfindungsgemäß müssen mindestens zwei Katalysatorlagen ausgebildet werden, wobei eine Katalysatorlage eine einheitliche Schüttung von Katalysatorkörpern im Reaktor darstellt. Die Ausdehnung der Schüttung im Reaktor in axialer Richtung bzw. in Gasdurchflussrichtung entspricht dabei der Länge $L_x$ der jeweiligen Katalysatorlage x. Sofern der Reaktor als senkrecht angeordnetes Rohr ausgebildet ist, ist die Länge der jeweiligen Katalysatorlage gleichbedeutend mit der Füllhöhe der jeweiligen Katalysatorlage. Die Zählung der x-ten Katalysatorlage erfolgt von der Gaseintrittsseite in Gasdurchflussrichtung. Um die Reaktion durchzuführen, werden durch den Reaktor z.B. stündlich von oben nach unten etwa 2 bis 5 Nm$^3$ Luft mit einer Beladung von 30 bis 100 g ortho-Xylol/Nm$^3$ Luft bei einem Gesamtdruck von ca. 1,2 - 1,6 bar geleitet. Generell können die aufeinander folgenden Katalysatorlagen unmittelbar aufeinander folgen, d.h. in Kontakt stehen, oder aber, z.B. durch eine Lage Inertkörper, voneinander getrennt vorliegen.

Die erste Katalysatorlage ist der Gaseintrittsseite des Reaktors zugewandt, daran anschließend in Gasdurchflussrichtung befindet sich die mindestens zweite Katalysatorlage, bestehend aus Katalysatorkörpern, die sich von den Katalysatorkörpern der ersten Katalysatorlage unterscheiden. Erfindungsgemäß weist die erste Katalysatorlage einen höheren Lückengrad auf als die zweite Katalysatorlage.

Die erste Katalysatorlage kann eine Länge von kleiner 1 m, bevorzugterweise von 10 bis 50 cm aufweisen. Es wird bevorzugt, dass die erste Katalysatorlage einen Längenanteil von 5 bis 25 %, besonders bevorzugt 15 bis 25 % der Rohrlänge L aufweist.

Die zweite Katalysatorlage kann eine Länge von 0,3 bis 3 m bevorzugterweise von 0,85 bis 2 m, besonders bevorzugterweise 1 bis 2 m aufweisen. Es wird bevorzugt, dass die zweite Katalysatorlage einen Längenanteil von 15 bis 60 %, insbesondere 20 bis 60 % oder 30 bis 50 % der Rohrlänge L aufweist.

An die zweite Katalysatorlage können sich in Gasdurchflussrichtung weitere Katalysatorlagen anschließen, z.B. eine dritte Katalysatorlage, die sich unmittelbar an die zweite Katalysatorlage anschließt, und möglicherweise eine vierte Katalysatorlage, die sich unmittelbar an die dritte Katalysatorlage anschließt. Eine entsprechende fünfte Katalysatorlage ist in der Regel nicht erforderlich, jedoch möglich.

Die dritte Katalysatorlage kann eine Länge von kleiner 1 m, bevorzugterweise 50 bis 70 cm aufweisen. Es wird bevorzugt, dass die dritte Katalysatorlage einen Längenanteil von etwa 10 bis 30 % der Rohrlänge L einnimmt, insbesondere wenn es sich bei der dritten Katalysatorlage um die letzte, also die dem Reaktorausgang am nächsten gelegene Katalysatorlage handelt, ist ein Längenanteil für die dritte Katalysatorlage von 20 bis 50 % der Rohrlänge L bevorzugt.

Die vierte Katalysatorlage kann eine Länge von 30 bis 150 cm, bevorzugterweise 55 bis 75 cm aufweisen. Es wird bevorzugt, dass eine solche vierte Katalysatorlage einen Längenanteil von etwa 10 bis 40 %, insbesondere bevorzugt 10 bis 30 % der Rohrlänge L einnimmt, insbesondere wenn es sich bei der vierten Katalysatorlage um die letzte, also die dem Reaktorausgang am nächsten gelegene Katalysatorlage handelt, ist ein Längenanteil an der Rohrlänge L für die vierte Katalysatorlage von 15 bis 25 % bevorzugt.

In einer weiteren besonders bevorzugten Ausführungsform ist der Längenanteil an der Rohrlänge L der ersten Katalysatorlage zwischen 15 bis 25 %, der zweiten Katalysatorlage zwischen 20 bis 50 %, der dritten Katalysatorlage zwischen 20 bis 50 % und der vierten Katalysatorlage zwischen 15 bis 25 %.

Der Lückengrad LG als Eigenschaft einer Katalysatorlage berechnet sich nach Gleichung 1 (Gl.1).

$$\text{Gl.1:} \qquad LG = 100 * (V_{KL} - V_{FK}) / V_{KL} = 100 * V_{LR} / V_{KL}$$

$V_{KL}$ = Gesamtvolumen der Katalysatorlage im Reaktor, d.h. $\pi * D^2/4 * L_x$ ($L_x$= Länge der jeweiligen Katalysatorlage x in Gasdurchflussrichtung, D= Innendurchmesser Reaktor)

$V_{LR}$ = Volumen des Lehrraums innerhalb der gefüllten Katalysatorlage

$V_{FK}$ = Volumen der Katalysatorkörper innerhalb der gefüllten Katalysatorlage

Um den höheren Lückengrad in der ersten Katalysatorlage auszubilden, können sich die Katalysatorkörper der ersten und der zweiten Katalysatorlage in einer oder mehreren geometrischen Abmessungen und / oder in ihrer geometrischen Form unterscheiden. Als geometrische Form bevorzugt sind die zylinderförmige und die ringförmige Form. Die geometrischen Abmessungen, entsprechend z.B. der Höhe, der Länge und der Breite des Katalysatorkörpers, sind bevorzugterweise so gewählt, dass sich ein Volumen des Katalysatorkörpers im Bereich von 0,05 bis 0,5 cm$^3$ ergibt.

Im einfachsten Fall weisen die Katalysatorkörper der zweiten Katalysatorlage dieselbe geometrische Form auf, wie die der ersten Katalysatorlage, sind aber kleiner, d.h. weisen geringere geometrische Abmessungen auf. Bevorzugt ist die Verwendung von Ringen als Katalysatorkörper, die in der zweiten Katalysatorlage kleiner sind, als in der ersten Katalysatorlage. Der verringerte Lückengrad kann aber auch durch Gemische von Katalysatorkörpern realisiert werden.

Der Lückengrad in der ersten Katalysatorlage kann zwischen 62 und 70 %, bevorzugterweise zwischen 64 und 68 % und insbesondere zwischen 65 und 66,5 % liegen. Das Verhältnis zwischen Länge und Lückengrad (= 100 * ($L_x$ / $LG_x$) [m]) kann für die erste Katalysatorlage zwischen 0,3 m bis 1,2 m, bevorzugterweise zwischen 0,5 bis 0,7 m betragen. Die Katalysatorkörper der ersten Katalysatorlage weisen vorzugsweise ein Verhältnis Oberfläche / Volumen von 15 bis 17 cm$^{-1}$ auf.

Der Lückengrad in der zweiten Katalysatorlage kann bei 60 bis 68 %, bevorzugterweise bei 62 bis 66 % liegen, unter der Voraussetzung, dass der Lückengrad der zweiten Katalysatorlage kleiner ist, als der der ersten Katalysatorlage. Insbesondere kann der Lückengrad zwischen 63 und weniger als 65 % betragen. Das Verhältnis zwischen Länge und Lückengrad (= 100 * ($L_x$ / $LG_x$) [m]) kann für die zweite Katalysatorlage zwischen 1,2 bis 4,8 m, bevorzugterweise zwischen 2,2 und 2,6 m betragen. Die Katalysatorkörper der zweiten Katalysatorlage weisen vorzugsweise ein Verhältnis Oberfläche/Volumen größer 17 bis 30 cm$^{-1}$ auf.

Vorzugsweise unterscheidet sich das Verhältnis Oberfläche/Volumen zwischen den Katalysatorkörpern der ersten Katalysatorlage und den Katalysatorkörpern der zweiten Katalysatorlage um mehr als 5 %, stärker bevorzugt um mehr als 7 %.

Schließt sich an die zweite Katalysatorlage eine dritte Katalysatorlage an, so ist bevorzugt, dass die dritte Katalysatorlage Katalysatorkörper aufweist, die einen Lückengrad aufweisen, der unterschiedlich bzw. höher ist als derjenige der zweiten Katalysatorlage. Der Lückengrad in der dritten Katalysatorlage kann zwischen 62 und 70 %, bevorzugterweise zwischen 64 und 68 % und insbesondere zwischen 65 und 66,5 % liegen, unter der Voraussetzung, dass der Lückengrad der dritten Katalysatorlage größer ist, als der der zweiten Katalysatorlage. Insbesondere ist bevorzugt, dass die dritte Katalysatorlage Katalysatorkörper aufweist, die denselben Lückengrad ausbilden wie die Katalysatorkörper der ersten Katalysatorlage. Dies wird bevorzugterweise dadurch realisiert, dass die Katalysatorkörper der dritten Katalysatorlage dieselbe geometrische Form und dieselben geometrischen Abmessungen aufweisen wie die der ersten Katalysatorlage. Das Verhältnis zwischen Länge und Lückengrad (= 100 * $L_x$ / $LG_x$ [m]) kann für die dritte Katalysatorlage zwischen 0,45 bis 1,80 m, bevorzugterweise zwischen 0,7 bis 1,1 m betragen.

Schließt sich an die dritte Katalysatorlage eine vierte Katalysatorlage an, so ist bevorzugt, dass die vierte Katalysatorlage Katalysatorkörper aufweist, die denselben Lückengrad ausbilden wie die Katalysatorkörper der ersten Katalysatorlage. Dies wird bevorzugterweise dadurch realisiert, dass die Katalysatorkörper der vierten Katalysatorlage dieselbe geometrische Form und dieselben geometrischen Abmessungen aufweisen wie die der ersten Katalysatorlage. Das Verhältnis zwischen Länge und Lückengrad (= 100 * $L_x$ / $LG_x$ [m]) kann für die vierte Katalysatorlage zwischen 0,5 bis 1,8 m, bevorzugterweise 0,8 bis 1,2 m betragen.

Die Katalysatorkörper der unterschiedlichen Katalysatorlagen können sich auch im Aktivmassegehalt unterscheiden. Gemäß einer Ausführungsform weisen die Katalysatorkörper einen Aktivmassegehalt zwischen 3 bis 12 Gew.-%, bevorzugt zwischen 4 bis 10 Gew.-% auf, bezogen auf das Gesamtgewicht der Katalysatorkörper. Bevorzugt ist, dass der Aktivmassegehalt der ersten Katalysatorlage höher ist, als der der zweiten Katalysatorlage. Z.B. kann der Aktivmassegehalt der Katalysatorkörper der ersten Katalysatorlage bei 7 bis 9 Gew.-% liegen und der der zweiten Katalysatorlage bei 4 bis 7 Gew.-%, jeweils bezogen auf das Gesamtgewicht der Katalysatorkörper. Bezüglich der möglicherweise folgenden dritten und vierten Katalysatorlage ist bevorzugt, dass sich der Aktivmassegehalt $m_A$ von der zweiten zur vierten Katalysatorlage erhöht, oder sich von der zweiten auf die dritte Katalysatorlage erhöht und von der dritten auf die vierte Katalysatorlage gleichbleibt.

Die Aktivmasse kann neben Vanadium zahlreiche Promotoren wie beispielsweise Alkali- und / oder Erdalkalimetalle, Antimon, Phosphor, Eisen, Niob, Kobalt, Molybdän, Silber, Wolfram, Zinn, Blei, Zirkonium, Kupfer, Gold, und/oder Wismut sowie Mischungen aus zwei oder mehreren der vorstehenden Komponenten haben. Die Katalysatorkörper der einzelnen Lagen unterscheiden sich in einer Ausführungsform in der Zusammensetzung ihrer Aktivmasse. Tabelle 1 gibt einen Überblick zur typischen Zusammensetzung der Aktivmasse.

**Tabelle 1: Typische Zusammensetzung der Aktivmasse**

| Komponente | Typischer Gehalt* |
|---|---|
| $V_2O_5$ | 0,5 bis 30 Gew.-%, insbesondere 1 bis 30 Gew.-% |
| $Sb_2O_3$ oder $Sb_2O_5$ | 0 bis 10 Gew.-% |
| Cs | 0 bis 2 Gew.-% |
| P | 0 bis 5 Gew.-% |
| Nb | 0 bis 5 Gew.-% |
| Weitere Komponenten wie Li, Na, K, Ba, W, Mo, Y, Ce, Mg, Sn, Bi, Fe, Ag, Co, Ni, Cu, Au, etc. | 0 bis 5 Gew.-% |
| $TiO_2$ | Rest zu 100 Gew.-% |
| *Die Prozentangaben beziehen sich jeweils auf das Gesamtgewicht der Aktivmasse. | |

Die Aktivmasse enthält vorzugsweise 5 bis 15 Gew.-% $V_2O_5$, 0 bis 5 Gew.-% $Sb_2O_3$, 0,2 bis 0,75 Gew.-% Cs, 0 bis 5 Gew.-% $Nb_2O_5$, jeweils bezogen auf das Gesamtgewicht der Aktivmasse. Neben den vorstehenden Komponenten besteht der Rest der Aktivmasse ganz oder im Wesentlichen aus $TiO_2$. Eine solche Aktivmasse kann beispielsweise vorteilhaft in den Katalysatorkörpern der ersten oder zweiten Katalysatorlage verwendet werden.

Bevorzugterweise weisen die Katalysatorkörper abhängig von der Katalysatorlage unterschiedlich zusammengesetzte Aktivmassen auf und/oder die Aktivmassen der einzelnen Katalysatorlagen unterscheiden sich in den physikalisch-chemischen Eigenschaften. Nach einer Ausführungsform liegt die BET-Oberfläche des Katalysators bzw. der Aktivmasse zwischen 15 und etwa 30 $m^2$/g. Die Aktivmasse kann aber z.B. in den Katalysatorkörpern abhängig von der Katalysatorlage jeweils eine unterschiedliche BET-Oberfläche aufweisen.

Nach einer bevorzugten Ausführungsform nimmt dabei die BET-Oberfläche der Aktivmasse der ersten Katalysatorlage zur Aktivmasse der vierten Katalysatorlage zu. Geeignete Bereiche für die BET-Oberfläche sind beispielsweise 15 bis 25 $m^2$/g für die erste Katalysatorlage, 15 bis 30 $m^2$/g für die zweite Katalysatorlage, 15 bis 30 $m^2$/g für die dritte Katalysatorlage und 20 bis 45 $m^2$/g für die vierte Katalysatorlage.

Nach einer besonders bevorzugten Ausführungsform enthält die Aktivmasse der Katalysatorkörper der ersten Katalysatorlage zwischen 5 bis 16 Gew.-% $V_2O_5$, 0 bis 5 Gew.-% $Sb_2O_3$, 0,2 bis 0,75 Gew.-% Cs, 0 bis 1 Gew.-% P und 0 bis 3 Gew.-% $Nb_2O_5$, jeweils bezogen auf das Gesamtgewicht der Aktivmasse. Der Rest der Aktivmasse besteht zu mindestens 90 Gew.-%, vorzugsweise mindestens 95 Gew.-%, weiter bevorzugt mindestens 98 Gew.-%, insbesondere mindestens 99 Gew.-%, weiter bevorzugt mindestens 99,5 Gew.-%, insbesondere 100 Gew.-% aus $TiO_2$. Nach einer besonders bevorzugten Ausführungsform liegt dabei die BET-Oberfläche des $TiO_2$ zwischen 15 und etwa 25 $m^2/g$.

Nach einer besonders bevorzugten Ausführungsform enthält die Aktivmasse der Katalysatorkörper der zweiten Katalysatorlage zwischen 5 bis 15 Gew.-% $V_2O_5$, 0 bis 5 Gew.-% $Sb_2O_3$, 0,2 bis 0,75 Gew.-% Cs, 0 bis 1 Gew.-% P und 0 bis 2 Gew.-% $Nb_2O_5$, jeweils bezogen auf das Gesamtgewicht der Aktivmasse. Der Rest der Aktivmasse besteht zu mindestens 90 Gew.-%, vorzugsweise mindestens 95 Gew.-%, weiter bevorzugt mindestens 98 Gew.-%, insbesondere mindestens 99 Gew.-%, weiter bevorzugt mindestens 99,5 Gew.-%, insbesondere 100 Gew.-% aus $TiO_2$. Nach einer besonders bevorzugten Ausführungsform liegt dabei die BET-Oberfläche des $TiO_2$ zwischen 15 und etwa 45 $m^2/g$.

Nach einer besonders bevorzugten Ausführungsform enthält die Aktivmasse der Katalysatorkörper der dritten Katalysatorlage zwischen 5 bis 15 Gew.-% $V_2O_5$, 0 bis 4 Gew.-% $Sb_2O_3$, 0,05 bis 0,5 Gew.-% Cs, 0 bis 1 Gew.-% P und 0 bis 2 Gew.-% $Nb_2O_5$, jeweils bezogen auf das Gesamtgewicht der Aktivmasse. Der Rest der Aktivmasse besteht zu mindestens 90 Gew.-%, vorzugsweise mindestens 95 Gew.-%, weiter bevorzugt mindestens 98 Gew.-%, insbesondere mindestens 99 Gew.-%, weiter bevorzugt mindestens 99,5 Gew.-%, insbesondere 100 Gew.-% aus $TiO_2$. Dabei wird bevorzugt, dass das $TiO_2$ eine BET-Oberfläche zwischen 15 und 25 $m^2/g$ aufweist.

Nach einer besonders bevorzugten Ausführungsform enthält die Aktivmasse des Katalysators der vierten Katalysatorlage zwischen 5 bis 25 Gew.-% $V_2O_5$, 0 bis 5 Gew.-% $Sb_2O_3$, 0 bis 0,2 Gew.-% Cs, 0 bis 2 Gew.-% P und 0 bis 1 Gew.-% $Nb_2O_5$, jeweils bezogen auf das Gesamtgewicht der Aktivmasse. Der Rest der Aktivmasse besteht zu mindestens 90 Gew.-%, vorzugsweise mindestens 95 Gew.-%, weiter bevorzugt mindestens 98 Gew.-%, insbesondere mindestens 99 Gew.-%, weiter bevorzugt mindestens 99,5 Gew.-%, insbesondere 100 Gew.-% aus $TiO_2$. Soweit die vierte Katalysatorlage die an der Gasaustrittsseite des Reaktors gelegene (letzte) Katalysatorlage darstellt, wird dabei eine BET-Oberfläche des $TiO_2$ bevorzugt, die etwas höher liegt, als diejenige des $TiO_2$ der näher zur Gaseintrittsseite hin gelegenen Katalysatorlagen. Es wird bevorzugt, dass das $TiO_2$ der Aktivmasse der vierten Katalysatorlage im Bereich zwischen etwa 15 bis etwa 45 $m^2/g$ liegt.

Weiterhin können sich die Katalysatorkörper der einzelnen Katalysatorlagen in ihrer intrinsischen Aktivität unterscheiden, die unterschiedliche intrinsische Aktivität kann sich zum Beispiel durch eine unterschiedliche Zusammensetzung der Aktivmasse ergeben. Erfindungsgemäß ist es bevorzugt, dass die Katalysatorkörper der ersten Katalysatorlage eine höhere intrinsische Aktivität aufweisen als die Katalysatorkörper der zweiten Katalysatorlage.

Unter der intrinsischen Aktivität eines Katalysatorkörpers $K_b$ wird die Aktivität der Aktivmasse für eine bestimmte Reaktion unter Bedingungen verstanden, unter denen die Reaktion an einem infinitesimal kleinen Katalysatorpartikel abläuft und die Reaktion durch die Nachbarschaft unbeeinflusst ist. Solche Bedingungen würden einem Temperaturgradienten über den Reaktor von 0 °C, einer unendlichen Raum-Zeit-Geschwindigkeit des Gases und einer unendlichen Entfernung zwischen den infinitesimal kleinen Katalysatorpartikeln entsprechen. Eine solche intrinsische Aktivität lässt sich beispielsweise durch eine Versuchsreihe ermitteln, die es ermöglicht, die Aktivität des Katalysatorkörpers in einem Zustand zu extrapolieren, der einem solchen idealen Zustand entspricht (z.B. Extrapolation der Versuchsergebnisse gegen einen Umsatz von Null).

Bei der praktischen Durchführung der Ermittlung der intrinsischen Aktivität wird gemäß einer Ausführungsform so vorgegangen, dass ein Katalysatorkörper mit einer bestimmten Geometrie und einem bestimmten Gehalt an Aktivmasse hergestellt wird. Ferner wird angenommen, dass die Reaktion erster Ordnung abläuft, unabhängig von der Ordnung, mit welcher die Reaktion tatsächlich abläuft. Dieser standardisierte Katalysatorkörper wird dann mit inerten Körpern so weit verdünnt, dass die Temperaturdifferenz zwischen der Gaseintritts- und der Gasaustrittsseite geringer als 25 °C, bevorzugt geringer als 10 °C ist, die Reaktion also unter nahezu isothermen Bedingungen abläuft, wobei der Druckabfall über den Reaktor weniger als 30 mbar, bevorzugt weniger als 10 mbar beträgt und wobei der Umsatz auf einen Wert im Bereich von 65 bis 95 % eingestellt wird.

Dazu wird der Katalysatorkörper mit Inertkörpern verdünnt. Die Geometrie der Katalysatorkörper und der Inertkörper wird so gewählt, dass der geforderte geringe Druckabfall verwirklicht wird. Das Verhältnis von Inertkörpern zu Katalysatorkörpern wird so gewählt, dass der geforderte Umsatz erreicht wird und gleichzeitig die Wärmeentwicklung so gering ist, dass die geforderte geringe Temperaturdifferenz zwischen Gaseintritt und Gasaustritt eingehalten wird. Bezogen auf das Volumen, welches sich aus der Schüttdichte der Katalysatorkörper bzw. der Inertkörper ergibt, wird bevorzugt ein Verhältnis von Katalysatorkörpern zu Inertkörpern von 1 : 5 bis 1 : 10 gewählt. Die Abmessungen des Testreaktors werden je nach der betrachteten Reaktion zwischen 1 und 6 m für die Länge und zwischen 18 und 32 mm für den Durchmesser des Reaktors gewählt. Bei schnellen Reaktionen wird eine kurze Länge gewählt, während für langsam ablaufende Reaktionen eine größere Reaktionsstrecke benötigt wird, um die geforderten Umsätze zu erreichen.

Es wird dann bei bestimmten Raum-Zeit-Geschwindigkeiten und bei verschiedenen bestimmten Temperaturen der Umsatz der Reaktion gemessen, die von der Aktivmasse des Katalysatorkörpers katalysiert wird. Aus den Umsätzen lässt sich dann die aktivmassenbezogene Aktivitätskonstante A* des Katalysators in Abhängigkeit vom Umsatz nach Gl. 2 berechnen:

Gl. 2:

$$A^* = \frac{[\text{GHSV} \times -1 \times \ln(1 - U)]}{[m_{\text{Aktivmasse}}]}$$

Hierbei bedeutet:

A*: Aktivmassenbezogene Aktivitätskonstante der Aktivmasse bei einer bestimmten Temperatur und GHSV;

GHSV: Raum-Zeitgeschwindigkeit [$h^{-1}$]

$m_{\text{Aktivmasse}}$: Menge der im Reaktor eingebrachten Aktivmasse [g];

U: Umsatz des Edukts, wobei sich U nach Gl. 3 berechnet.

Gl. 3:

$$U = \frac{M_{\text{rein}} - M_{\text{raus}}}{M_{\text{rein}}}$$

$M_{\text{rein}}$: Menge an Edukt [mol] welches der Katalysatorfüllung zugeführt wird

$M_{\text{raus}}$: Menge an Edukt [mol] welches die Katalysatorfüllung verlässt

Aus den ermittelten aktivmassenbezogenen Aktivitätskonstanten A* als Funktion des Umsatzes U wird dann durch lineare Extrapolation gegen einen Umsatz von Null die intrinsische Aktivität $A_{ib}$ des Katalysators bestimmt. Die für die Extrapolation einzustellenden Umsätze werden so gewählt, dass diese im linearen Bereich der Abhängigkeit der aktivmassenbezogenen Aktivitätskonstante vom Umsatz liegen (z.B. Umsatz zwischen 60 % und 90 %).

Während des Betriebs der erfindungsgemäßen Katalysatoranordnung bildet sich ein Temperaturprofil aus. Bevorzugt ist dabei, dass das Temperaturprofil derart ist, dass bei der Gasphasenoxidation von aromatischen Kohlenwasserstoffen die Maximaltemperatur in der ersten Katalysatorlage um 10 bis 100 °C, mehr bevorzugt 20 bis 90 °C, am meisten bevorzugt 30 bis 70 °C niedriger ist als in der zweiten Katal ysatorlage.

Ist eine dritte Katalysatorlage vorhanden, so ist die intrinsische Aktivität der dritten Katalysatorlage vorzugsweise höher, als die der zweiten Katalysatorlage. Vorzugsweise entsteht während der Reaktion ein von der zweiten Katalysatorlage zur dritten Katalysatorlage abfallendes Temperaturprofil. Insbesondere ist das Temperaturprofil derart, dass bei der Gasphasenoxidation von Kohlenwasserstoffen die Maximaltemperatur in der dritten Katalysatorlage um 10 bis 100 °C, mehr bevorzugt 20 bis 90 °C, am meisten bevorzugt 30 bis 70 °C niedriger ist als in der zweiten Katalysatorlage.

Ist eine vierte Katalysatorlage vorhanden, so ist die intrinsische Aktivität der vierten Katalysatorlage vorzugsweise höher, als die der dritten Katalysatorlage. Dabei ist vorzugsweise das Temperaturprofil von der zweiten zur vierten (und auch zur dritten) Katalysatorlage abfallend ($T_2 > T_3 > T_4$, jeweils bezogen auf die Maximaltemperatur in jeder Lage). Insbesondere ist das Temperaturprofil derart, dass bei der Gasphasenoxidation von Kohlenwasserstoffen die Maximaltemperatur in der vierten Katalysatorlage um 10 bis 100 °C, mehr bevorzugt 20 bis 90 °C, am meisten bevorzugt 30 bis 70 °C niedriger ist als in der zweiten und um 1 bis 50 °C, mehr bevorzugt um 5 bis 25°C, am meisten bevorzugt um 5 bis 10°C niedriger ist als in der dritten Katalysatorlage.

Bevorzugt ist insbesondere, dass die intrinsische Aktivität von der ersten zur zweiten Katalysatorlage abfällt und dann von der zweiten zur vierten Katalysatorlage ($A_1 > A_2 < A_3 < A_4$, mit A = intrinsische Aktivität der Aktivmasse in der jeweiligen Lage) ansteigt. Weiterhin bevorzugt ist, dass das Temperaturprofil von der ersten zur zweiten Katalysatorlage ansteigt, von der zweiten zur vierten Katalysatorlage abfällt ($T_1 < T_2 > T_3 > T_4$, jeweils bezogen auf die Maximaltemperatur in jeder Lage) und sich ein Temperaturmaximum in der zweiten Katalysatorlage ausbildet.

Die Katalysatorkörper bestehen aus einem inerten Trägerkörper und einer darauf aufgebrachten Aktivmasse. Die Katalysatorkörper der Katalysatorlagen werden in der üblichen Weise hergestellt, wobei eine dünne Schicht der Aktivmasse auf den inerten Trägerkörper aufgebracht wird. Dazu kann beispielsweise eine Suspension der Aktivmasse oder eine Lösung bzw. Suspension von Vorläuferverbindungen, die in die Komponenten der Aktivmasse überführt werden können, auf den inerten Träger aufgesprüht werden. Dies kann beispielsweise bei einer Temperatur von 80 bis 200 °C im Fli eßbett erfolgen. Die Aktivmasse kann aber beispielsweise auch in einer Art Dragiertrommel auf den inerten Träger aufgebracht werden.

Für den Beschichtungsvorgang wird die wässrige Lösung oder Suspension von Aktivkomponenten und eines organischen Binders, bevorzugterweise einem Co-Polymer aus Vinylacetat/Vinyllaurat, Vinylacetat/Ethylen oder Styrol/Acrylat, über eine oder mehrere Düsen auf den erwärmten, fluidisierten Träger aufgesprüht. Besonders günstig ist es, die Sprühflüssigkeit am Ort der höchsten Produktgeschwindigkeit aufzugeben, wodurch sich der Sprühstoff gleichmäßig im Bett verteilen kann. Der Sprühvorgang wird solange fortgeführt, bis entweder die Suspension verbraucht oder die erforderliche Menge an Aktivkomponenten auf dem Träger aufgebracht ist. Unter Aktivkomponenten werden Komponente der Aktivmasse verstanden, insbesondere in der Aktivmasse enthaltene Metallverbindungen. Die Aktivkomponenten können als Oxide oder in Form von Vorläuferverbindungen eingesetzt werden. Unter Vorläuferverbindungen werden Verbindungen verstanden, die sich beispielsweise durch Erhitzen an Luft in die Komponenten der Aktivmasse, also die Oxide, überführen lassen. Geeignete Vorläuferverbindungen sind beispielsweise Nitrate, Sulfate, Karbonate, Acetate oder Chloride der Metalle.

Nach einer Ausführungsform wird die Aktivmasse im Fließbett oder Wirbelbett unter Beihilfe geeigneter Bindemittel aufgebracht, so dass ein Schalenkatalysator erzeugt wird. Geeignete Bindemittel umfassen dem Fachmann geläufige organische Binder, bevorzugt Co-Polymere, vorteilhaft in Form einer wässrigen Suspensionen von Vinylacetat/Vinyllaurat, Vinylacetat/Acrylat, Styrol, Acrylat, Vinylacetat/Maleat sowie Vinylacetat/Ethylen. Besonders bevorzugt wird ein organischer polymerer oder co-polymerer Kleber, insbesondere ein Vinylacetat-Copolymer-Kleber als Bindemittel verwendet. Das verwendete Bindemittel wird in den üblichen Mengen der Aktivmasse zugegeben, beispielsweise mit etwas 10 bis 20 Gew.-% bezogen auf den Feststoffgehalt der Aktivmasse. Beispielhaft wird auf die EP 744214 verwiesen.

Die Bestimmung des Binderanteils wird durchgeführt, indem die beschichteten Katalysatorkörper bei 450 °C für 7 h kalziniert werden, wobei sich der organische Binder vollständig thermisch zersetzt. Der Binderanteil wird im Anschluss an die Kalzinierung nach Gl. 4 bestimmt:

$$\text{Gl. 4:} \qquad A_B = \frac{M_E - M_A}{M_E} * 100\%$$

$A_B$ = Binderanteil
$M_E$ = Einwaage Katalysator vor Kalzinierung
$M_A$ = Auswaage Katalysator nach Kalzinierung

Die physikalisch-chemische Charakterisierung der Aktivmasse (BET, chemische Analytik) wird durchgeführt, indem nach der thermischen Zersetzung des Binders, die Aktivmasse mechanisch mittels eines Siebes von den Trägerringen abgetrennt wird. Der restliche, noch an den Trägerringen anhaftende Teil der Aktivmasse wurde durch Ultraschallbehandlung vollständig entfernt. Abschließend wurden die gewaschenen Trägerringe bei 120 °C in einem Trockenschrank getrocknet und gewogen. Der Anteil der Aktivmasse wird im Anschluss nach Gl. 5 bestimmt:

$$\text{Gl. 5:} \qquad A_A = \frac{M_A - M_T}{M_A} * 100\%$$

$A_A$ = Anteil Aktivmasse
$M_A$ = Auswaage Katalysator nach Kalzinierung
$M_T$ = Auswaage Trägerringe

Die Bestimmung der spezifischen Oberfläche der Materialien erfolgt nach der BET-Methode gemäss DIN 66131; eine Veröffentlichung der BET-Methode findet sich auch in J. Am. Chem. Soc. 60, 309 (1938). Die zu bestimmende Probe wurde in einem Quarzrohr bei 350 °C unter Vakuum getrocknet (F = 50 ml(min) für 1,5 h). Der Reaktor wurde dann auf Raumtemperatur abgekühlt, evakuiert und in ein Dewar-Gefäß mit flüssigem Stickstoff getaucht. Die Stickstoff-Adsorption wurde bei 77 K mit einem RXM 100 Sorptionssystem (Advanced Scientific Design, Inc.) durchgeführt.

Beispiele

Es wurden katalytische Messungen an 4-Lagen-Katalysatoranordnungen aus Katalysatorkörpern durchgeführt. Zur Synthese der Katalysatorkörper wurden drei verschiedene Typen Steatitringe als Formkörper mit der Bezeichnung Ring 8x6x5, Ring 7x7x4 und Ring 6x5x4 verwendet. Die Nomenklatur der geometrischen Abmessungen der Ringe entspricht Außendurchmesser (Da) [mm] x Höhe (H) [mm] x Innendurchmesser (Di) [mm]. Die geometrischen Abmessungen der unbeschichteten Formkörper ergeben sich aus Tabelle 2. Die unbeschichteten Formkörper wurden in eine Beschichtungsapparatur eingebracht und homogen mit der Aktivmasse beschichtet. Während des Beschichtungsvorgangs wird eine wässrige Suspension der Aktivkomponenten und eines organischen Binders über mehrere Düsen auf den erwärmten, fluidisierten Träger aufgesprüht, bis sich eine Aktivmasseschicht von ca. 50 - 150 $\mu$m ausgebildet hat. Tabelle 3 zeigt eine Übersicht über die verwendeten Katalysatorkörper und die jeweilige Zusammensetzung der Aktivmasse.

Der Lückengrad LG der Katalysatorlagen wurde mit Gl. 6 berechnet.

Gl. 6:

$$LG = \left[1 - \frac{\rho_{Sch\"utt}}{\rho_{Kat}}\right] * 100\%$$

$\rho_{Sch\"utt}$ = Schüttdichte der Katalysatorkörper [g/cm$^3$]
$\rho_{Kat}$ = Scheinbare Dichte der Katalysatorkörper [g/cm$^3$] (ca. 2,5 g/cm$^3$)
LG = Lückengrad [%]

Zur Ermittlung der Schüttdichte wurde ein Rohr, das im Durchmesser annähernd dem Innendurchmesser des Reaktorrohrs entspricht (Innendurchmesser 2,7 cm), mit dem entsprechenden Katalysator jeweils auf 100,0 cm Füllhöhe aufgefüllt. Um ein gleichmäßiges Füllen zu gewährleisten, wurden die Katalysatorkörper vereinzelt in das Rohr gegeben. Ein gleichmäßiges Füllen konnte visuell überwacht werden, da ein transparentes Kunststoffrohr verwendet wurde. Nach erfolgreicher Füllung wurde das Rohr vollständig entleert und die Masse der eingefüllten Katalysatorkörper mittels einer Waage bestimmt. Die Schüttdichte der Katalysatorkörper ergab sich hieraus nach folgender Gl. 7:

Gl. 7:

$$\rho_{Sch\"utt} = \frac{m_{Kat}}{\pi \frac{d^2}{4} h}$$

$\rho_{Sch\"utt}$:      Schüttdichte Katalysatorkörper [g/cm$^3$]
$m_{Kat}$:      Masse eingefüllter Katalysatorkörper [g]
d:      Innendurchmesser Rohr [cm] = 2,7 cm
h:      Füllhöhe Rohr [cm] = 100,0 cm

Die Schüttdichte wurde für jeden Typ Katalysatorkörper dreimal bestimmt, um hieraus den im Weiteren verwendeten arithmetischen Mittelwert zu berechnen.

Die scheinbare Dichte ("Bulk-Density") der Katalysatorkörper entspricht dem Quotient aus der Gesamtmasse des Katalysatorkörpers (d.h. inklusive der Masse des Steatitrings und der aufgetragene Aktivmasse) und dem Gesamtvolumen des Formkörpers unter Berücksichtigung der äußeren Oberfläche. Das Gesamtvolumen des Formkörpers entspricht hierbei dem Volumen des Formkörpers, inklusive dem Volumen eventuell vorhandener Poren. Die scheinbare Dichte wurde mit Hilfe eines Quecksilber-Porosimeters bestimmt, dank der Eigenschaft des Quecksilbers, die Wände einer Feststoffprobe nicht zu benetzen, wird Druck benötigt, um mögliche Poren in der Probe zu durchdringen. Dadurch lässt sich das Gesamtvolumen bestimmen, denn steht das System Probe + Quecksilber unter Vakuum, so kann Quecksilber nicht in das Innere der Poren eindringen und bedeckt dann ausschließlich die externe Oberfläche.

Die scheinbare Dichte der unterschiedlichen Katalysatorkörper aus Tabelle 3 variiert um Werte < 0,05 g/cm$^3$.

Zur Ausbildung der Katalysatorlagen wurden die jeweiligen Katalysatorkörper in ein salzbadgekühltes Rohr mit 25 mm innerem Durchmesser und 4 m Länge gefüllt. Im Rohr befand sich zentrisch angeordnet eine 3 mm Thermohülse mit eingebautem Zugelement zur Temperaturmessung.

Zur Durchführung der katalytischen Messung wurde durch das Rohr stündlich von oben nach unten etwa 3,7 bis 4,0 Nm$^3$ (Normkubikmeter) Luft mit einer Beladung von 30 bis 100 g or-tho-Xylol/Nm$^3$ Luft (Reinheit ortho-Xylol > 98 %) bei einem Gesamtdruck von ca. 1500 mbar geleitet. Die Messungen wurden jeweils bei einer Beladung von etwa 40 bis 100 g ortho-Xylol/Nm$^3$ Luft und einer Salzbadtemperaturen zwischen 350 und 390 °C durchgeführt.

Die Phthalsäureanhydrid-Ausbeute wurde mit Gl. 8 berechnet:

Gl. 8:      $$Y_{PA} = \left[139,52 - \left(800 * \frac{A+B}{E}\right) + (100 - F) - (1,25 * H) - (1,1 * G)\right]$$

A = CO$_2$ im Produktstrom [Vol.-%]

B = CO im Produktstrom [Vol.-%]

H = Maleinsäureanhydrid im Produktstrom [Gew.-%]

E = ortho-Xylol-Beladung im Eduktstrom [g/Nm$^3$/h/Rohr]

F = ortho-Xylol-Reinheit des eingesetzten ortho-Xylols [Gew.-%]

G = ortho-Xylol-Schlupf in Bezug auf das gesamte eingesetzte ortho-Xylol [Gew.-%]

$Y_{PA}$ = Ausbeute Phthalsäureanhydrid (PSA) bezogen auf das Gesamtgewicht des eingesetzten ortho-Xylols [Gew.-%]

Wie aus Gl. 8 ersichtlich, ist die Phthalsäureanhydrid-Ausbeute unmittelbar abhängig von der Bildung der drei wichtigsten Nebenprodukte CO, $CO_2$ und Maleinsäureanhydrid.

**Tabelle 2: Geometrische Abmessungen und Eigenschaften der unbeschichteten Formkörper**

|  | Ring 8x6x5 | Ring 7x7x4 | Ring 6x5x4 |
|---|---|---|---|
| Außendurchmesser x Höhe [cm$^2$] | 0,48 | 0,28 | 0,30 |
| Volumen [cm$^3$] | 0,184 | 0,104 | 0,079 |
| Oberfläche [cm$^2$] | 3,063 | 1,901 | 1,885 |
| Oberfläche/Volumen [cm$^{-1}$] | 16,7 | 18,3 | 24,0 |
| Volumen/Oberfläche [cm] | 0,060 | 0,055 | 0,042 |
| scheinbare Dichte [g/cm$^3$] | 2,61 | 2,61 | 2,61 |

**Tabelle 3: Verwendete Katalysatorkörper**

| Bezeichnung | Ringform (Da x H x Di)[1] [mm] | Binderanteil [Gew.-%][3] | Anteil Aktivmasse [Gew.-%][2] | BET [m²/g] | TiO$_2$ [Gew.-%][3] | V$_2$O$_5$ [Gew.-%][3] | Promotoren [Gew.-%][4] |
|---|---|---|---|---|---|---|---|
| Vergleichstest 1 | | | | | | | |
| A0 | 8x6x5 | 2,3 | 8,5 | 18 | 87,4 | 7,5 | 5,1 |
| A1 | 8x6x5 | 2,3 | 8,5 | 18 | 87,4 | 7,5 | 5,1 |
| A2 | 8x6x5 | 2,3 | 8,0 | 18 | 89,0 | 7,5 | 3,5 |
| A3 | 8x6x5 | 2,3 | 8,0 | 27 | 90,6 | 9,0 | 0,4 |
| Vergleichstest 2 | | | | | | | |
| B0 | 8x6x5 | 2,4 | 8,3 | 19 | 87,4 | 7,5 | 5,1 |
| B1 | 8x6x5 | 2,4 | 8,6 | 18 | 87,3 | 7,6 | 5,1 |
| B2.1 | 8x6x5 | 2,4 | 8,0 | 18 | 89,1 | 7,4 | 3,5 |
| B3.1 | 8x6x5 | 2,2 | 7,9 | 26 | 90,6 | 9,1 | 0,4 |
| Erfindungsgemäßer Test 1 | | | | | | | |
| B1 | 8x6x5 | 2,4 | 8,6 | 18 | 87,3 | 7,6 | 5,1 |
| D1 | 6x5x4 | 2,3 | 5,3 | 19 | 84,7 | 7,2 | 8,1 |
| B2.1 | 8x6x5 | 2,4 | 8,0 | 18 | 89,1 | 7,4 | 3,5 |
| B3.2 | 8x6x5 | 2,4 | 7,9 | 25 | 90,2 | 9,4 | 0,4 |
| Erfindungsgemäßer Test 2 | | | | | | | |
| B0 | 8x6x5 | 2,4 | 8,3 | 19 | 87,4 | 7,5 | 5,1 |
| E1 | 6x5x4 | 2,2 | 5,3 | 24 | 83,7 | 10,7 | 5,6 |
| B2.1 | 8x6x5 | 2,4 | 8,0 | 18 | 89,1 | 7,4 | 3,5 |
| B3.2 | 8x6x5 | 2,4 | 7,9 | 25 | 90,2 | 9,4 | 0,4 |
| Erfindungsgemäßer Test 3 | | | | | | | |
| B0 | 8x6x5 | 2,4 | 8,3 | 19 | 87,4 | 7,5 | 5,1 |
| F1 | 7x7x4 | 2,2 | 5,3 | 27 | 83,0 | 10,6 | 5,6 |
| B2.2 | 8x6x5 | 2,4 | 8,0 | 18 | 88,0 | 7,4 | 3,5 |

(fortgesetzt)

| Bezeichnung | Ringform | Binderanteil | Anteil Aktivmasse | BET | $TiO_2$ | $V_2O_5$ | Promotoren |
|---|---|---|---|---|---|---|---|
| | (Da x H x Di)[1] [mm] | [Gew.-%][3] | [Gew.-%][2] | [m$^2$/g] | [Gew.-%][3] | [Gew.-%][3] | [Gew.-%][4] |
| B3.1 | 8x6x5 | 2,2 | 7,9 | 26 | 90,6 | 9,1 | 0,4 |

[1] Da = Außendurchmesser, H = Höhe, Di = Innendurchmesser
[2] Bezogen auf das Gesamtgewicht des Katalysatorkörpers
[3] Bezogen auf das Gesamtgewicht der Aktivmasse
[4] Vorwiegend $Sb_2O_3$ neben geringeren Anteilen, $Nb_2O_5$, P und Cs

**Tabelle 4: Füllparameter des Vergleichstests 1**

| Katalysatorlage x | Katalysator | Füllhöhen $L_x$ | Schüttdichte | scheinbare Dichte | Lückengrad, LG | Ringform |
|---|---|---|---|---|---|---|
| | | [cm] | [g/cm$^3$] | [g/cm$^3$] | [%] | (Da x H x Di) [mm] |
| 1 | A0 | 40,9 | 0,92 | 2,623 | 65,1 | 8x6x5 |
| 2 | A1 | 160,0 | 0,91 | 2,664 | 65,8 | 8x6x5 |
| 3 | A2 | 60,9 | 0,91 | 2,622 | 65,3 | 8x6x5 |
| 4 | A3 | 59,5 | 0,91 | 2,663 | 65,8 | 8x6x5 |

**Tabelle 5: Füllparameter des Vergleichstests 2**

| Katalysatorlage x | Katalysator | Füllhöhen $L_x$ | Schüttdichte | scheinbare Dichte | Lückengrad, LG | Ringform |
|---|---|---|---|---|---|---|
| | | [cm] | [g/cm$^3$] | [g/cm$^3$] | [%] | (Da x H x Di) [mm] |
| 1 | B0 | 40,0 | 0,89 | 2,624 | 66,0 | 8x6x5 |
| 2 | B1 | 135,3 | 0,91 | 2,610 | 65,2 | 8x6x5 |
| 3 | B2.1 | 60,5 | 0,90 | 2,619 | 65,8 | 8x6x5 |
| 4 | B3.1 | 64,9 | 0,92 | 2,643 | 65,2 | 8x6x5 |

**Tabelle 6: Füllparameter des erfindungsgemäßen Tests 1**

| Katalysatorlage x | Katalysator | Füllhöhen $L_x$ | Schüttdichte | scheinbare Dichte | Lückengrad, LG | Ringform |
|---|---|---|---|---|---|---|
| | | [cm] | [g/cm$^3$] | [g/cm$^3$] | [%] | (Da x H x Di) [mm] |
| 1 | B1 | 39,8 | 0,84 | 2,610 | 65,2 | 8x6x5 |
| 2 | D1 | 154,5 | 0,91 | 2,631 | 62,6 | 6x5x4 |
| 3 | B2.1 | 60,5 | 0,84 | 2,619 | 65,8 | 8x6x5 |
| 4 | B3.2 | 64,8 | 0,84 | 2,620 | 65,0 | 8x6x5 |

**Tabelle 7: Füllparameter des erfindungsgemäßen Tests 2**

| Katalysatorlage x | Katalysator | Füllhöhen $L_x$ | Schüttdichte | scheinbare Dichte | Lückengrad, LG | Ringform |
|---|---|---|---|---|---|---|
| | | [cm] | [g/cm$^3$] | [g/cm$^3$] | [%] | (Da x H x Di) [mm] |
| 1 | B0 | 39,7 | 0,89 | 2,624 | 66,0 | 8x6x5 |
| 2 | E1 | 155,3 | 0,96 | 2,693 | 64,5 | 6x5x4 |
| 3 | B2.1 | 60,9 | 0,90 | 2,619 | 65,8 | 8x6x5 |
| 4 | B3.2 | 64,3 | 0,92 | 2,620 | 65,0 | 8x6x5 |

**Tabelle 8: Füllparameter des erfindungsgemäßen Tests 3**

| Katalysatorlage x | Katalysator | Füllhöhen $L_x$ | Schüttdichte | Scheinbare Dichte | Lückengrad, LG | Ringform |
|---|---|---|---|---|---|---|
| | | [cm] | [g/cm$^3$] | [g/cm$^3$] | [%] | (Da x H x Di) [mm] |
| 1 | B0 | 41,0 | 0,89 | 2,62 | 66,0 | 8x6x5 |
| 2 | F1 | 155,0 | 0,93 | 2,60 | 64,1 | 7x7x4 |
| 3 | B2.2 | 60,5 | 0,88 | 2,57 | 65,7 | 8x6x5 |
| 4 | B3.1 | 64,5 | 0,92 | 2,64 | 65,2 | 8x6x5 |

**Tabelle 9: Katalysatorperformance des Vergleichstests 1**

| TOS | Volumenstrom Luft | Beladung ortho-Xylol | SBT | CO | $CO_2$ | MSA | ortho-Xylol | Ausbeute PSA | (CO+$CO_2$)/ Beladung |
|---|---|---|---|---|---|---|---|---|---|
| [h] | [$Nm^3$] | [g/$Nm^3$/h/ Rohr] | [°C] | [Vol.-%][1] | [Vol.-%][1] | [Gew.-%][2] | [Gew.-%][2] | [Gew.-%][3] | [Vol.-%/g/$Nm^3$/h/Rohr] |
| 178,6 | 4,0 | 33,7 | 379 | 0,337 | 0,781 | 5,0 | 0,01 | 108,7 | 0,0332 |
| 203,4 | 4,0 | 35,7 | 378 | 0,357 | 0,827 | 5,1 | 0,01 | 108,7 | 0,0332 |
| 705,1 | 4,0 | 46,3 | 371 | 0,465 | 1,115 | 4,9 | 0,02 | 108,0 | 0,0341 |
| 728,7 | 4,0 | 48,3 | 370 | 0,487 | 1,193 | 5,0 | 0,02 | 107,5 | 0,0348 |
| 752,2 | 4,0 | 48,3 | 370 | 0,490 | 1,137 | 5,0 | 0,02 | 108,3 | 0,0337 |
| 2821,4 | 4,0 | 79,9 | 359 | 0,749 | 1,785 | 4,5 | 0,03 | 110,5 | 0,0317 |
| 2847,0 | 4,0 | 79,9 | 360 | 0,752 | 1,798 | 4,5 | 0,03 | 110,3 | 0,0319 |
| 2867,2 | 4,0 | 81,4 | 360 | 0,757 | 1,810 | 4,5 | 0,03 | 110,7 | 0,0315 |

**Tabelle 10: Katalysatorperformance des Vergleichstests 2**

| TOS | Volumenstrom Luft | Beladung ortho-Xylol | SBT | CO | $CO_2$ | MSA | ortho-Xylol | Ausbeute PSA | (CO+$CO_2$)/ Beladung |
|---|---|---|---|---|---|---|---|---|---|
| [h] | [$Nm^3$] | [g/$Nm^3$/h/Rohr] | [°C] | [Vol.-%][1] | [Vol.-%][1] | [Gew.-%][2] | [Gew.-%][2] | [Gew.-%][3] | [Vol.-%/g/$Nm^3$/h/Rohr] |
| 254,3 | 3,7 | 73,1 | 368 | 0,758 | 1,712 | 4,9 | 0,02 | 108,4 | 0,0338 |
| 278,4 | 3,7 | 73,1 | 368 | 0,732 | 1,671 | 4,7 | 0,02 | 109,3 | 0,0328 |
| 302,5 | 3,7 | 73,1 | 368 | 0,726 | 1,670 | 4,7 | 0,02 | 109,5 | 0,0328 |
| 538,1 | 3,7 | 82,0 | 364 | 0,806 | 1,866 | 4,7 | 0,02 | 109,6 | 0,0326 |
| 562,1 | 3,7 | 83,9 | 363 | 0,820 | 1,958 | 4,7 | 0,03 | 109,2 | 0,0331 |
| 761,3 | 3,7 | 90,9 | 356 | 0,848 | 2,048 | 4,3 | 0,02 | 110,6 | 0,0319 |
| 785,3 | 3,7 | 90,9 | 356 | 0,848 | 2,056 | 4,3 | 0,03 | 110,6 | 0,0320 |
| 809,3 | 3,7 | 90,9 | 356 | 0,838 | 2,038 | 4,2 | 0,03 | 110,9 | 0,0316 |

[1] Bezogen auf das Gesamtvolumen des Produktstroms
[2] Bezogen auf das Gesamtgewicht des Produktstroms
[3] Bezogen auf das Gesamtgewicht des eingesetzten Xylols

**Tabelle 11: Katalysatorperformance des erfindungsgemäßen Tests 1**

| TOS | Volumenstrom Luft | Beladung ortho-Xylol | SBT | CO | $CO_2$ | MSA | ortho-Xylol | Ausbeute PSA | (CO+$CO_2$)/ Beladung |
|---|---|---|---|---|---|---|---|---|---|
| [h] | [$Nm^3$] | [g/$Nm^3$/h/Rohr] | [°C] | [Vol.-%][1] | [Vol.-%][1] | [Gew.-%][2] | [Gew.-%][2] | [Gew.-%][3] | [Vol.-%/g/$Nm^3$/h/Rohr] |
| 318,6 | 4,0 | 38,9 | 382 | 0,390 | 0,830 | 4,1 | 0,01 | 111,3 | 0,0313 |
| 342,5 | 4,0 | 39,9 | 380 | 0,398 | 0,854 | 4,0 | 0,01 | 111,5 | 0,0313 |
| 532,7 | 4,0 | 45,9 | 372 | 0,436 | 0,958 | 3,7 | 0,01 | 112,6 | 0,0304 |
| 557,9 | 4,0 | 48,1 | 370 | 0,447 | 0,989 | 3,7 | 0,01 | 113,1 | 0,0299 |
| 579,6 | 4,0 | 50,1 | 369 | 0,458 | 1,033 | 3,6 | 0,02 | 113,2 | 0,0298 |
| 680,8 | 4,0 | 54,0 | 365 | 0,479 | 1,089 | 3,6 | 0,02 | 113,8 | 0,0290 |
| 698,9 | 4,0 | 56,0 | 364 | 0,494 | 1,136 | 3,6 | 0,02 | 113,8 | 0,0291 |
| 916,5 | 4,0 | 63,9 | 362 | 0,530 | 1,321 | 3,6 | 0,03 | 113,8 | 0,0289 |

[1] Bezogen auf das Gesamtvolumen des Produktstroms
[2] Bezogen auf das Gesamtgewicht des Produktstroms
[3] Bezogen auf das Gesamtgewicht des eingesetzten Xylols

**Tabelle 12: Katalysatorperformance des erfindungsgemäßer Test 2**

| TOS [h] | Volumenstrom Luft [Nm³] | Beladung ortho-Xylol [g/Nm³/h/Rohr] | SBT [°C] | CO [Vol.-%][1] | $CO_2$ [Vol.-%][1] | MSA [Gew.-%][2] | ortho-Xylol [Gew.-%][2] | Ausbeute PSA [Gew.-%][3] | (CO+$CO_2$)/ Beladung [Vol.-%/g/Nm³/h/Rohr] |
|---|---|---|---|---|---|---|---|---|---|
| 156,2 | 4,0 | 43,0 | 387 | 0,463 | 1,019 | 4,6 | 0,01 | 108,2 | 0,0345 |
| 180,4 | 4,0 | 47,0 | 384 | 0,508 | 1,115 | 4,7 | 0,01 | 108,0 | 0,0345 |
| 204,3 | 4,0 | 51,0 | 381 | 0,560 | 1,230 | 4,8 | 0,01 | 107,5 | 0,0351 |
| 229,5 | 4,0 | 55,9 | 377 | 0,595 | 1,317 | 4,7 | 0,01 | 108,3 | 0,0342 |
| 250,5 | 4,0 | 59,9 | 373 | 0,581 | 1,318 | 4,3 | 0,02 | 110,7 | 0,0317 |
| 274,5 | 4,0 | 59,9 | 373 | 0,587 | 1,332 | 4,3 | 0,02 | 110,5 | 0,0320 |
| 298,5 | 4,0 | 59,9 | 373 | 0,585 | 1,335 | 4,3 | 0,02 | 110,5 | 0,0321 |
| 348,5 | 4,0 | 66,1 | 367 | 0,578 | 1,408 | 3,9 | 0,03 | 112,6 | 0,0300 |
| 1260,8 | 4,0 | 78,0 | 357 | 0,636 | 1,585 | 3,3 | 0,08 | 114,5 | 0,0285 |
| 1284,9 | 4,0 | 78,0 | 357 | 0,633 | 1,539 | 3,2 | 0,07 | 115,2 | 0,0279 |
| 1309,0 | 4,0 | 78,0 | 357 | 0,623 | 1,511 | 3,2 | 0,07 | 115,6 | 0,0274 |
| 1335,1 | 4,0 | 80,9 | 357 | 0,636 | 1,587 | 3,2 | 0,09 | 115,5 | 0,0275 |
| 1383,6 | 4,0 | 84,9 | 356 | 0,676 | 1,650 | 3,5 | 0,10 | 115,2 | 0,0274 |
| 1406,4 | 4,0 | 84,9 | 355 | 0,681 | 1,663 | 3,5 | 0,09 | 115,0 | 0,0276 |
| 1551,6 | 4,0 | 84,9 | 355 | 0,677 | 1,731 | 3,5 | 0,13 | 114,4 | 0,0284 |
| 1578,4 | 4,0 | 84,9 | 355 | 0,648 | 1,747 | 3,5 | 0,12 | 114,5 | 0,0282 |
| 1621,3 | 4,0 | 84,9 | 355 | 0,674 | 1,717 | 3,5 | 0,13 | 114,5 | 0,0282 |

**Tabelle 13: Katalysatorperformance des erfindungsgemäßen Tests 3**

| TOS | Volumenstrom Luft | Beladung ortho-Xylol | SBT | CO | $CO_2$ | MSA | ortho-Xylol | Ausbeute PSA | (CO+$CO_2$)/ Beladung |
|---|---|---|---|---|---|---|---|---|---|
| [h] | [Nm$^3$] | [g/Nm$^3$/h/Rohr] | [°C] | [Vol.-%][1] | [Vol.-%][1] | [Gew.-%][2] | [Gew.-%][2] | [Gew.-%][3] | [Vol.-%/g/Nm$^3$/h/Rohr] |
| 164,3 | 4,0 | 40,6 | 386 | 0,465 | 0,900 | 4,5 | 0,01 | 109,0 | 0,0336 |
| 184,2 | 4,0 | 43,2 | 382 | 0,480 | 0,944 | 4,5 | 0,01 | 109,5 | 0,0330 |
| 207,6 | 4,0 | 46,2 | 378 | 0,511 | 1,072 | 4,3 | 0,02 | 108,7 | 0,0343 |
| 231,6 | 4,0 | 46,2 | 378 | 0,506 | 1,060 | 4,3 | 0,02 | 109,0 | 0,0339 |
| 255,6 | 4,0 | 46,2 | 378 | 0,506 | 1,070 | 4,3 | 0,00 | 108,8 | 0,0341 |
| 279,6 | 4,0 | 51,2 | 374 | 0,559 | 1,197 | 4,3 | 0,02 | 108,8 | 0,0343 |
| 309,8 | 4,0 | 55,3 | 370 | 0,567 | 1,229 | 4,2 | 0,03 | 110,3 | 0,0325 |
| 333,8 | 4,0 | 61,3 | 367 | 0,637 | 1,417 | 4,1 | 0,03 | 109,6 | 0,0335 |
| 351,5 | 4,0 | 65,4 | 365 | 0,657 | 1,470 | 4,2 | 0,04 | 110,2 | 0,0325 |
| 387,6 | 4,0 | 71,4 | 363 | 0,705 | 1,608 | 4,1 | 0,03 | 110,4 | 0,0324 |
| 404,3 | 4,0 | 71,4 | 363 | 0,684 | 1,559 | 3,9 | 0,03 | 111,5 | 0,0314 |
| 428,3 | 4,0 | 71,4 | 363 | 0,671 | 1,534 | 3,9 | 0,03 | 111,9 | 0,0309 |
| 452,3 | 4,0 | 79,5 | 362 | 0,793 | 1,839 | 4,2 | 0,04 | 109,8 | 0,0331 |
| 474,4 | 4,0 | 82,5 | 362 | 0,814 | 1,902 | 4,2 | 0,04 | 109,9 | 0,0329 |
| 498,4 | 3,9 | 87,9 | 361 | 0,870 | 2,053 | 4,2 | 0,05 | 109,6 | 0,0333 |
| 522,4 | 3,9 | 87,9 | 360 | 0,855 | 2,032 | 4,1 | 0,05 | 110,0 | 0,0329 |
| 546,3 | 3,9 | 89,9 | 359 | 0,849 | 2,028 | 4,0 | 0,07 | 110,8 | 0,0320 |
| 570,4 | 3,9 | 89,9 | 359 | 0,846 | 2,039 | 4,0 | 0,07 | 110,8 | 0,0321 |
| 618,4 | 3,9 | 93,9 | 358 | 0,891 | 2,171 | 4,0 | 0,11 | 110,3 | 0,0326 |

[1] Bezogen auf das Gesamtvolumen des Produktstroms
[2] Bezogen auf das Gesamtgewicht des Produktstroms
[3] Bezogen auf das Gesamtgewicht des eingesetzten ortho-Xylols

**EP 3 008 050 B1**

**Patentansprüche**

1. Katalysatoranordnung zur Herstellung von Phthalsäureanhydrid durch Gasphasenoxidation aromatischer Kohlenwasserstoffe, umfassend einen Reaktor mit einer Gaseintrittsseite für ein Eduktgas und einer Gasaustrittsseite für ein Produktgas, sowie einer ersten Katalysatorlage aus Katalysatorkörpern und mindestens einer zweiten Katalysatorlage aus Katalysatorkörpern, wobei die erste Katalysatorlage an der Gaseintrittsseite und die zweite Katalysatorlage nachfolgend zur ersten Katalysatorlage in Gasdurchflussrichtung angeordnet ist und wobei die Länge der ersten Katalysatorlage in Gasdurchflussrichtung kleiner ist als die Länge in Gasdurchflussrichtung der zweiten Katalysatorlage, **dadurch gekennzeichnet, dass** die erste Katalysatorlage im Vergleich zur zweiten Katalysatorlage einen höheren Lückengrad aufweist.

2. Katalysatoranordnung nach Anspruch 1, **dadurch gekennzeichnet, dass** der höhere Lückengrad dadurch entsteht, dass sich die Katalysatorkörper der ersten Katalysatorlage von den Katalysatorkörpern der zweiten Katalysatorlage in einer oder mehreren geometrischen Abmessung(en) und/oder in ihrer geometrischen Form unterscheiden.

3. Katalysatoranordnung nach Anspruch 2, **dadurch gekennzeichnet, dass** die Katalysatorkörper der ersten Katalysatorlage und die Katalysatorkörper der zweiten Katalysatorlage ringförmig sind und die Katalysatorkörper der zweiten Katalysatorlage eine geringere geometrische Abmessung aufweisen als die der ersten Katalysatorlage.

4. Katalysatoranordnung nach einem der vorhergehenden Ansprüche, **dadurch gekennzeichnet, dass** der Lückengrad der ersten Katalysatorlage um mindestens 0,6 % höher ist als der Lückengrad der zweiten Katalysatorlage.

5. Katalysatoranordnung nach einem der vorhergehenden Ansprüche, **dadurch gekennzeichnet, dass** der Lückengrad der ersten Katalysatorlage um mindestens 1,5 % höher ist als der Lückengrad der zweiten Katalysatorlage.

6. Katalysatoranordnung nach einem der vorhergehenden Ansprüche, **dadurch gekennzeichnet, dass** die Katalysatorkörper der ersten Katalysatorlage jeweils bezogen auf die Masse der Katalysatorkörper eine höhere Aktivmassebeladung aufweisen als die Katalysatorkörper der zweiten Katalysatorlage.

7. Katalysatoranordnung nach einem der vorhergehenden Ansprüche, **dadurch gekennzeichnet, dass** die Aktivmasse der Katalysatorkörper der ersten Katalysatorlage eine niedrigere BET-Oberfläche aufweist als die Aktivmasse der Katalysatorkörper der zweiten Katalysatorlage.

8. Katalysatoranordnung nach einem der vorhergehenden Ansprüche, **dadurch gekennzeichnet, dass** die Katalysatorkörper der ersten Katalysatorlage eine Aktivmasse mit einem auf die Masse der Aktivmasse bezogenen prozentual niedrigeren $V_2O_5$-Gehalt aufweisen als die Katalysatorkörper der zweiten Katalysatorlage.

9. Katalysatoranordnung nach einem der vorhergehenden Ansprüche, **dadurch gekennzeichnet, dass** die Katalysatorkörper der ersten Katalysatorlage eine Aktivmasse mit einem auf die Masse der Aktivmasse bezogenen prozentual niedrigeren Promotor-Gehalt aufweisen als die Katalysatorkörper der zweiten Katalysatorlage.

10. Katalysatoranordnung nach einem der vorhergehenden Ansprüche, **dadurch gekennzeichnet, dass** eine dritte Katalysatorlage nachfolgend zur zweiten Katalysatorlage in Gasdurchflussrichtung angeordnet ist, die im Vergleich zur zweiten Katalysatorlage einen höheren Lückengrad aufweist.

11. Katalysatoranordnung nach Anspruch 10, **dadurch gekennzeichnet, dass** die Katalysatorkörper der zweiten Katalysatorlage bezogen auf die Masse der Katalysatorkörper eine niedrigere Aktivmassebeladung aufweisen als die Katalysatorkörper der dritten Katalysatorlage.

12. Katalysatoranordnung nach einem der Ansprüche 10 oder 11, **dadurch gekennzeichnet, dass** die Katalysatorkörper der zweiten Katalysatorlage eine Aktivmasse mit einer höheren BET-Oberfläche aufweisen als die der dritten Katalysatorlage.

13. Katalysatoranordnung nach einem der Ansprüche 10 bis 12, **dadurch gekennzeichnet, dass** die Katalysatorkörper der zweiten Katalysatorlage eine Aktivmasse mit einem auf die Masse der Aktivmasse bezogenen prozentual höheren $V_2O_5$-Gehalt aufweisen als die der dritten Katalysatorlage.

14. Katalysatoranordnung nach einem der Ansprüche 10 bis 13, **dadurch gekennzeichnet, dass** die Katalysatorkörper

20

der zweiten Katalysatorlage eine Aktivmasse mit einem auf die Masse der Aktivmasse bezogenen höheren Promotor-Gehalt aufweisen als die der dritten Katalysatorlage.

15. Katalysatoranordnung nach einem der vorhergehenden Ansprüche, **dadurch gekennzeichnet, dass** die Länge der ersten Katalysatorlage in Gasdurchflussrichtung 5 bis 25 % der Länge des Reaktors in Gasdurchflussrichtung beträgt.

16. Katalysatoranordnung nach einem der Ansprüche 10 bis 14, **dadurch gekennzeichnet, dass** die Länge der zweiten Katalysatorlage in Gasdurchflussrichtung 30 bis 60 % der Länge des Reaktors in Gasdurchflussrichtung beträgt.

17. Verfahren zur Herstellung von Phthalsäureanhydrid durch Gasphasenoxidation aromatischer Kohlenwasserstoffe, **dadurch gekennzeichnet, dass** ein Eduktgas, das einen aromatischen Kohlenwasserstoff enthält, durch eine Katalysatoranordnung nach einem der vorhergehenden Ansprüche geleitet wird.

18. Verwendung einer Katalysatoranordnung nach einem der Ansprüche 1 bis 16 zur Herstellung von Phthalsäureanhydrid.

## Claims

1. Catalyst arrangement for preparing phthalic anhydride by gas-phase oxidation of aromatic hydrocarbons, which comprises a reactor having a gas inlet end for a feed gas and a gas outlet end for a product gas and also a first catalyst zone made up of catalyst bodies and at least one second catalyst zone made up of catalyst bodies, where the first catalyst zone is arranged at the gas inlet end and the second catalyst zone is arranged downstream of the first catalyst zone in the gas flow direction and the length of the first catalyst zone in the gas flow direction is less than the length of the second catalyst zone in the gas flow direction, **characterized in that** the first catalyst zone has a higher gap content compared to the second catalyst zone.

2. Catalyst arrangement according to Claim 1, **characterized in that** the higher gap content is due to the catalyst bodies of the first catalyst zone differing in terms of one or more geometric dimension(s) and/or their geometric shape from the catalyst bodies of the second catalyst zone.

3. Catalyst arrangement according to Claim 2, **characterized in that** the catalyst bodies of the first catalyst zone and the catalyst bodies of the second catalyst zone are ring-shaped and the catalyst bodies of the second catalyst zone have smaller geometric dimensions than those of the first catalyst zone.

4. Catalyst arrangement according to any of the preceding claims, **characterized in that** the gap content of the first catalyst zone is at least 0.6% higher than the gap content of the second catalyst zone.

5. Catalyst arrangement according to any of the preceding claims, **characterized in that** the gap content of the first catalyst zone is at least 1.5% higher than the gap content of the second catalyst zone.

6. Catalyst arrangement according to any of the preceding claims, **characterized in that** the catalyst bodies of the first catalyst zone have, in each case based on the mass of the catalyst bodies, a higher active composition loading than the catalyst bodies of the second catalyst zone.

7. Catalyst arrangement according to any of the preceding claims, **characterized in that** the active composition of the catalyst bodies of the first catalyst zone has a lower BET surface area than the active composition of the catalyst bodies of the second catalyst zone.

8. Catalyst arrangement according to any of the preceding claims, **characterized in that** the catalyst bodies of the first catalyst zone have an active composition having a lower percentage $V_2O_5$ content, based on the mass of the active composition, than the catalyst bodies of the second catalyst zone.

9. Catalyst arrangement according to any of the preceding claims, **characterized in that** the catalyst bodies of the first catalyst zone have an active composition having a lower percentage promoter content, based on the mass on the active composition, than the catalyst bodies of the second catalyst zone.

**10.** Catalyst arrangement according to any of the preceding claims, **characterized in that** a third catalyst zone which has a higher gap content compared to the second catalyst zone is arranged downstream of the second catalyst zone in the gas flow direction.

**11.** Catalyst arrangement according to Claim 10, **characterized in that** the catalyst bodies of the second catalyst zone have, based on the mass of the catalyst bodies, a lower active composition loading than the catalyst bodies of the third catalyst zone.

**12.** Catalyst arrangement according to either Claim 10 or 11, **characterized in that** the catalyst bodies of the second catalyst zone have an active composition having a higher BET surface area than those of the third catalyst zone.

**13.** Catalyst arrangement according to any of Claims 10 to 12, **characterized in that** the catalyst bodies of the second catalyst zone have an active composition having a higher percentage $V_2O_5$ content, based on the mass of the active composition, than those of the third catalyst zone.

**14.** Catalyst arrangement according to any of Claims 10 to 13, **characterized in that** the catalyst bodies of the second catalyst zone have an active composition having a higher promoter content, based on the mass of the active composition, than those of the third catalyst zone.

**15.** Catalyst arrangement according to any of the preceding claims, **characterized in that** the length of the first catalyst zone in the gas flow direction is from 5 to 25% of the length of the reactor in the gas flow direction.

**16.** Catalyst arrangement according to any of Claims 10 to 14, **characterized in that** the length of the second catalyst zone in the gas flow direction is from 30 to 60% of the length of the reactor in the gas flow direction.

**17.** Process for preparing phthalic anhydride by gas-phase oxidation of aromatic hydrocarbons, **characterized in that** a feed gas containing an aromatic hydrocarbon is passed through a catalyst arrangement according to any of the preceding claims.

**18.** Use of a catalyst arrangement according to any of Claims 1 to 16 for preparing phthalic anhydride.


**Revendications**

**1.** Dispositif catalytique destiné à la production d'anhydride de l'acide phtalique par oxydation en phase gazeuse d'hydrocarbures aromatiques, comprenant un réacteur avec un côté d'entrée de gaz pour un gaz d'éduit et un côté de sortie de gaz pour un gaz de produit, ainsi qu'avec une première couche de catalyseur en corps catalytiques et au moins une deuxième couche de catalyseur en corps catalytiques, dans lequel la première couche de catalyseur est disposée sur le côté d'entrée de gaz et la deuxième couche de catalyseur est disposée à la suite de la première couche de catalyseur dans la direction d'écoulement du gaz et dans lequel la longueur de la première couche de catalyseur dans la direction d'écoulement du gaz est plus petite que la longueur de la deuxième couche de catalyseur dans la direction d'écoulement du gaz, **caractérisé en ce que** la première couche de catalyseur présente un plus grand degré de vide que la deuxième couche de catalyseur.

**2.** Dispositif catalytique selon la revendication 1, **caractérisé en ce que** le plus grand degré de vide est créé par le fait que les corps catalytiques de la première couche de catalyseur se différencient des corps catalytiques de la deuxième couche de catalyseur en ce qui concerne une ou plusieurs dimension(s) géométrique(s) et/ou leur forme géométrique.

**3.** Dispositif catalytique selon la revendication 2, **caractérisé en ce que** les corps catalytiques de la première couche de catalyseur et les corps catalytiques de la deuxième couche de catalyseur sont de forme annulaire et les corps catalytiques de la deuxième couche de catalyseur présentent une dimension géométrique plus faible que ceux de la première couche de catalyseur.

**4.** Dispositif catalytique selon l'une quelconque des revendications précédentes, **caractérisé en ce que** le degré de vide de la première couche de catalyseur est d'au moins 0,6 % plus élevé que le degré de vide de la deuxième couche de catalyseur.

5. Dispositif catalytique selon l'une quelconque des revendications précédentes, **caractérisé en ce que** le degré de vide de la première couche de catalyseur est d'au moins 1,5 % plus élevé que le degré de vide de la deuxième couche de catalyseur.

6. Dispositif catalytique selon l'une quelconque des revendications précédentes, **caractérisé en ce que** les corps catalytiques de la première couche de catalyseur, respectivement rapportés à la masse des corps catalytiques, présentent une charge de masse active plus élevée que les corps catalytiques de la deuxième couche de catalyseur.

7. Dispositif catalytique selon l'une quelconque des revendications précédentes, **caractérisé en ce que** la masse active des corps catalytiques de la première couche de catalyseur présente une surface BET plus petite que la masse active des corps catalytiques de la deuxième couche de catalyseur.

8. Dispositif catalytique selon l'une quelconque des revendications précédentes, **caractérisé en ce que** les corps catalytiques de la première couche de catalyseur présentent une masse active avec une teneur en $V_2O_5$, rapportée à la masse de la masse active, plus faible en pourcentage que les corps catalytiques de la deuxième couche de catalyseur.

9. Dispositif catalytique selon l'une quelconque des revendications précédentes, **caractérisé en ce que** les corps catalytiques de la première couche de catalyseur présentent une masse active avec une teneur en promoteur, rapportée à la masse de la masse active, plus faible en pourcentage que les corps catalytiques de la deuxième couche de catalyseur.

10. Dispositif catalytique selon l'une quelconque des revendications précédentes, **caractérisé en ce qu'**une troisième couche de catalyseur est disposée à la suite de la deuxième couche de catalyseur dans la direction d'écoulement du gaz, qui présente un degré de vide plus élevé que la deuxième couche de catalyseur.

11. Dispositif catalytique selon la revendication 10, **caractérisé en ce que** les corps catalytiques de la deuxième couche de catalyseur présentent, rapportée à la masse des corps catalytiques, une charge de masse active moins élevée que les corps catalytiques de la troisième couche de catalyseur.

12. Dispositif catalytique selon une des revendications 10 ou 11, **caractérisé en ce que** les corps catalytiques de la deuxième couche de catalyseur présentent une plus grande surface BET que ceux de la troisième couche de catalyseur.

13. Dispositif catalytique selon l'une quelconque des revendications 10 à 12, **caractérisé en ce que** les corps catalytiques de la deuxième couche de catalyseur présentent une masse active avec une teneur en $V_2O_5$, rapportée à la masse de la masse active, plus élevée en pourcentage que ceux de la troisième couche de catalyseur.

14. Dispositif catalytique selon l'une quelconque des revendications 10 à 13, **caractérisé en ce que** les corps catalytiques de la deuxième couche de catalyseur présentent une masse active avec une teneur en promoteur, rapportée à la masse de la masse active, plus élevée que ceux de la troisième couche de catalyseur.

15. Dispositif catalytique selon l'une quelconque des revendications précédentes, **caractérisé en ce que** la longueur de la première couche de catalyseur dans la direction d'écoulement du gaz vaut de 5 à 25 % de la longueur du réacteur dans la direction d'écoulement du gaz.

16. Dispositif catalytique selon l'une quelconque des revendications 10 à 14, **caractérisé en ce que** la longueur de la deuxième couche de catalyseur dans la direction d'écoulement du gaz vaut de 30 à 60 % de la longueur du réacteur dans la direction d'écoulement du gaz.

17. Procédé de production d'anhydride de l'acide phtalique par oxydation en phase gazeuse d'hydrocarbures aromatiques, **caractérisé en ce que** l'on conduit un gaz d'éduit, qui contient un hydrocarbure aromatique, à travers un dispositif catalytique selon l'une quelconque des revendications précédentes.

18. Utilisation d'un dispositif catalytique selon l'une quelconque des revendications 1 à 16 pour la production d'anhydride de l'acide phtalique.

**IN DER BESCHREIBUNG AUFGEFÜHRTE DOKUMENTE**

**In der Beschreibung aufgeführte Patentdokumente**

- WO 9961434 A1
- WO 9961433 A1
- WO 2004103944 A1
- WO 2007134849 A1
- WO 2011032658 A1
- WO 2006092304 A1
- WO 2008077791 A1
- EP 0985648 A1
- EP 744214 A

**In der Beschreibung aufgeführte Nicht-Patentliteratur**

- *J. Am. Chem. Soc.,* 1938, vol. 60, 309